# DEMANDE DE BREVET EUROPEEN

(11) **EP 1 504 779 A1**
(43) Date de publication de la demande: **09.02.2005**
(21) Numéro de dépôt: 04291976.1
(22) Date de dépôt: 02.08.2004
(51) Int. Cl.: A61M 5/148

(54) **Enveloppe déformable pour dispositif d'injection**

(30) Priorité: 08.08.2003 FR 0309801
(71) Demandeur: Medex (S.A.), 74941 Annecy le Vieux Cedex (FR)
(72) Inventeur: Lacroix, Jean Pierre, 74940 Annecy le Vieux (FR)
(74) Mandataire: Gasquet, Denis

(57) **Abrégé**

Enveloppe déformable (1) pour un dispositif d'injection sous pression (50) d'un liquide (101) conditionné en poche souple (100), destinée à s'étendre à l'intérieur d'une enceinte (52) qui, d'une part, comporte une ouverture (53) apte à être obturée par des moyens de fermeture (54) formant couvercle, et qui, d'autre part, contient un liquide inerte (51) susceptible d'être mis en pression entre la surface interne (55) de l'enceinte (52) et la surface externe (3) de l'enveloppe déformable (1), ladite enveloppe déformable (1) comportant une ouverture (2) destinée à être solidarisée hermétiquement à proximité de l'ouverture (53) de l'enceinte (52) et à être obturée par les moyens de fermeture (54), caractérisée en ce que chaque portion de l'enveloppe déformable (1), qui est destinée à entrer en contact avec les moyens de fermeture (54), comporte une zone de renfort dont l'épaisseur est sensiblement supérieure à l'épaisseur du reste de ladite enveloppe déformable (1).

## Description

La présente invention concerne une enveloppe déformable destinée à un dispositif d'injection sous pression d'un liquide conditionné en poche souple.

L'invention trouve une application particulièrement avantageuse, mais non exclusive, dans le domaine de l'injection de liquide à usage médical.

En médecine, l'injection de liquide est couramment utilisée notamment lors d'opérations de transfusion, de perfusion, de nutrition artificielle par voie veineuse ou digestive, d'injection de liquide de contraste, etc. Parmi toutes les techniques connues, l'injection haute pression de liquide conditionné en poche souple constitue l'une des plus adaptée au milieu médical en raison de l'hygiène satisfaisante qu'elle procure.

On rappelle qu'une poche souple est schématiquement composée de deux feuilles en matériau flexible qui sont solidarisées par collage ou soudage au niveau de leurs pourtours respectifs, ou d'une gaine tubulaire, de façon à constituer un volume interne apte à recevoir un liquide à injecter. Un orifice d'écoulement est bien évidemment ménagé en périphérie.

Au cours de son utilisation, une telle poche souple est destinée à être reliée à différents éléments amovibles. On pense notamment ici à un perfuseur de remplissage, une canalisation intermédiaire, un conduit d'injection tel qu'un cathéter ou une aiguille hypodermique, etc. C'est pourquoi l'orifice d'écoulement de la poche souple est généralement connecté à un raccord de liaison par l'intermédiaire d'un conduit d'écoulement plus ou moins long ; ce raccord de liaison étant apte à être connecté de manière réversible à n'importe lequel des éléments amovibles précités.

Parmi les dispositifs d'injection sous pression connus de l'état de la technique, on distingue notamment ceux qui utilisent la mise en pression d'un liquide inerte pour comprimer, via une enveloppe déformable formant interface, une poche souple contenant un liquide à injecter.

Un tel dispositif d'injection comporte schématiquement une enceinte à l'intérieur de laquelle s'étend une enveloppe déformable dont l'ouverture est solidarisée hermétiquement à proximité de l'ouverture de ladite enceinte. L'enveloppe déformable ainsi positionnée définie, en combinaison avec la surface interne de l'enceinte, un volume variable dans lequel est contenu le liquide inerte. Le volume interne de l'enveloppe déformable est quant à lui destiné à recevoir la poche souple.

Un tel dispositif d'injection comporte en outre habituellement des moyens de fermeture, formant couvercle, qui sont en mesure d'obturer l'ouverture de l'enceinte, et conséquemment l'ouverture de l'enveloppe déformable du fait de la proximité desdites ouvertures. Ces moyens de fermeture, qui peuvent être composés de plusieurs éléments complémentaires, servent avant tout de moyens de fixation pour la poche souple. En effet, le maintien de cette dernière à l'intérieur de l'enceinte est généralement réalisé en coinçant le conduit d'écoulement dans un alésage ou une fente, de section légèrement inférieure, ménagé dans le couvercle de fermeture.

Ce type de dispositif d'injection sous pression présente toutefois l'inconvénient d'engendrer à l'usage une usure excessive de l'enveloppe déformable, notamment au niveau des portions de parois qui sont destinées à venir enserrer le conduit d'écoulement de la poche souple lorsque cette dernière est mise en place à l'intérieur de ladite enveloppe déformable. Or il s'avère que dans la pratique, cette usure excessive va irrémédiablement conduire à la perforation de la paroi de l'enveloppe déformable, et donc à une mise hors service du dispositif d'injection dans son ensemble.

De part sa solidarisation à proximité de l'ouverture de l'enceinte, la partie supérieure de l'enveloppe déformable va être en effet amenée à venir régulièrement au contact des moyens de fermeture lors des nombreuses opérations d'ouverture et de fermeture de l'enceinte, inhérentes à l'utilisation du dispositif d'injection. Or, que ce soit lors de la mise en place des moyens de fermeture ou lors de leur retrait, la paroi interne de l'enveloppe déformable subit à chaque fois d'importantes contraintes de frottement qui vont rapidement engendrer de l'usure. Il est vrai que comme tout matériau souple, la matière constituant l'enveloppe déformable présente une résistance relativement faible à l'abrasion. Quoi qu'il en soit, ce phénomène d'usure va entraîner plus ou moins rapidement un percement de l'enveloppe déformable, et donc une fuite du circuit de mise en pression du liquide inerte, rendant ainsi le dispositif d'injection inutilisable.

Aussi le problème technique à résoudre, par l'objet de la présente invention, est de proposer une enveloppe déformable pour un dispositif d'injection sous pression d'un liquide conditionné en poche souple, destinée à s'étendre à l'intérieur d'une enceinte qui d'une part comporte une ouverture apte à être obturée par des moyens de fermeture formant couvercle et qui d'autre part contient un liquide inerte susceptible d'être mis en pression entre la surface interne de l'enceinte et la surface externe de l'enveloppe déformable, ladite enveloppe déformable comportant une ouverture destinée à être solidarisée hermétiquement à proximité de l'ouverture de l'enceinte et à être obturée par les moyens de fermeture, enveloppe déformable qui permettrait d'éviter les problèmes de l'état de la technique en offrant notamment une durée d'utilisation sensiblement allongée.

La solution au problème technique posé consiste, selon la présente invention, en ce que chaque portion de l'enveloppe déformable, qui est destinée à entrer en contact avec les moyens de fermeture, comporte une zone de renfort dont l'épaisseur est sensiblement supérieure à l'épaisseur du reste de ladite enveloppe déformable.

L'invention telle qu'ainsi définie présente l'avantage d'offrir une capacité de résistance à l'usure sensiblement améliorée, aux endroits précis où se manifestent les frottements générés lors des mises en place et retraits répétés des moyens de fermeture. Car si elle n'est pas en mesure de réduire le phénomène d'usure à proprement parler, la surépaisseur de matière est par contre tout à fait à même de retarder considérablement une éventuelle perforation de la paroi de l'enveloppe déformable. Le but est de rendre la durée de vie de l'enveloppe déformable compatible avec celle du dispositif d'injection haute pression dans son ensemble.

Le fait que le renforcement de l'enveloppe déformable soit localisé uniquement dans les zones de frottements permet de ne pas remettre en cause la souplesse générale de l'enveloppe déformable. Cette dernière peut ainsi, au sein du dispositif d'injection, continuer à jouer pleinement son rôle d'interface flexible entre le liquide inerte et la poche souple contenant le liquide à injecter.

La présente invention concerne également les caractéristiques qui ressortiront au cours de la description qui va suivre, et qui devront être considérées isolément ou selon toutes leurs combinaisons techniques possibles.

Cette description donnée à titre d'exemple non limitatif, fera mieux comprendre comment l'invention peut être réalisée, en référence aux dessins annexés sur lesquels :
La figure 1 illustre un dispositif d'injection sous pression d'un liquide conditionné en poche souple, comportant une enveloppe déformable conforme à la présente invention.
La figure 2 représente le dispositif d'injection de la figure 1 en position ouverte, c'est-à-dire avant insertion de la poche souple contenant le liquide à injecter.
La figure 3 montre en perspective l'enveloppe déformable des figures 1 et 2.
La figure 4 fait apparaître l'enveloppe déformable de la figure 3 en vue de face.
La figure 5 est une coupe transversale de l'enveloppe déformable de la figure 4, suivant le plan AA.
La figure 6 constitue une coupe transversale de l'enveloppe déformable de la figure 4, suivant le plan BB.
La figure 7 montre une coupe longitudinale de l'enveloppe déformable de la figure 4, suivant le plan CC.
La figure 8 fait apparaître une coupe longitudinale de l'enveloppe déformable de la figure 4, suivant le plan DD.

Pour des raisons de clarté, les mêmes éléments ont été désignés par des références identiques. De même, seuls les éléments essentiels pour la compréhension de l'invention ont été représentés, et ceci sans respect de l'échelle et de manière schématique.

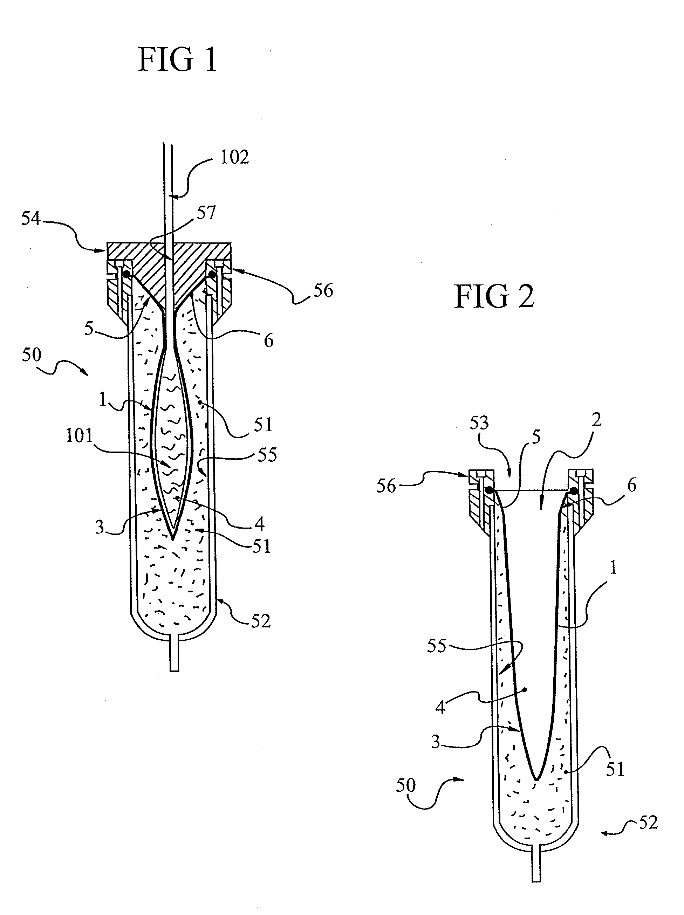

Les figures 1 et 2 font apparaître un dispositif d'injection haute pression (50) d'un liquide (101) conditionné en poche souple (100). Ce dispositif se singularise par le fait qu'il utilise une enveloppe déformable (1) pour former interface, entre un liquide inerte (51) susceptible d'être comprimé à l'intérieur d'une enceinte (52), d'une part, et la poche souple (100) contenant le liquide (101) à injecter, d'autre part.

L'enceinte (52), contenant le liquide inerte (51), est dotée, par ailleurs, d'une ouverture (53) qui est susceptible d'être obturée par des moyens de fermeture (54) amovibles, formant couvercle. L'enveloppe déformable (1), s'étendant à l'intérieur de l'enceinte (52), est quant à elle pourvue d'une ouverture (2) qui est solidarisée hermétiquement à proximité de l'ouverture (53) de l'enceinte (52), afin d'être également en mesure de coopérer par emboîtement avec les moyens de fermeture (54). Dans le cas présent, la liaison hermétique entre l'enveloppe déformable (1) et l'enceinte (52) est classiquement réalisée par coincement au moyen d'une bride (56) vissée à la partie supérieure de ladite enceinte (52).

L'ensemble est agencé de manière à ce que la surface externe (3) de l'enveloppe déformable (1) définisse, en combinaison avec la surface interne (55) de l'enceinte (52), un volume variable dans lequel est contenu le liquide inerte (51). Le volume interne (4) de l'enveloppe déformable (1) est quant à lui destiné à recevoir la poche souple (100). Il est à noter que le liquide inerte (51) est susceptible d'être mis en pression par des moyens externes qui n'ont pas été représentés ici pour des raisons de clarté.

Comme leurs homologues de l'art antérieur, les moyens de fermeture (54) sont à même d'obturer à la fois l'ouverture (53) de l'enceinte (51) et l'ouverture (2) de l'enveloppe déformable (1). De même, leur fonction principale demeure le maintien de la poche souple (100) à l'intérieur de l'enceinte (51). La fixation est ici réalisée classiquement en coinçant le conduit d'écoulement (102) de la poche souple (100) dans un alésage (57), de section légèrement inférieure, ménagé à travers le couvercle de fermeture (54).

Conformément à l'objet de la présente invention, chaque portion de l'enveloppe déformable (1), qui est destinée à entrer en contact avec les moyens de fermeture (54), comporte une zone de renfort (5) dont l'épaisseur est sensiblement supérieure à celle du reste de ladite enveloppe déformable (1). Cela signifie que chaque portion de paroi de l'enveloppe déformable (1), qui est destinée à subir des frottements lors de la mise en oeuvre des moyens de fermeture (54), présente une épaisseur sensiblement plus importante que l'épaisseur des portions de paroi de ladite enveloppe déformable (1) qui ne sont pas destinées à entrer en contact avec lesdits moyens de fermeture (54).

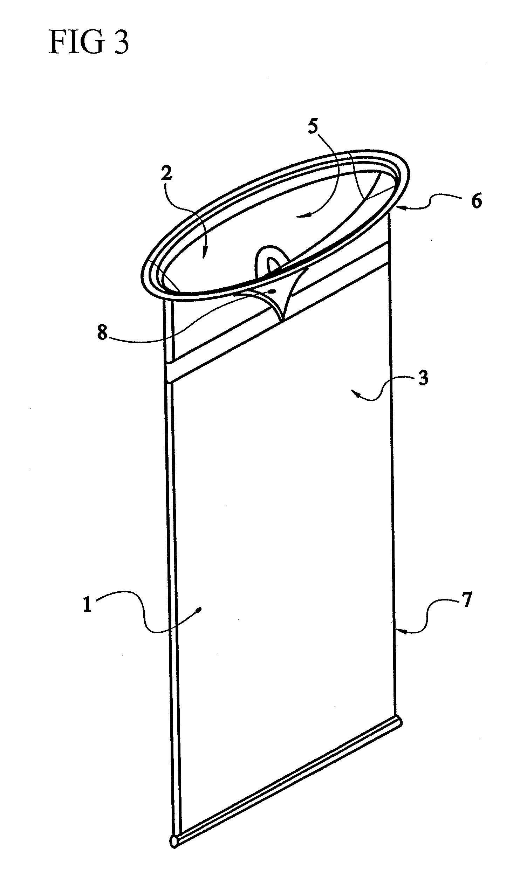

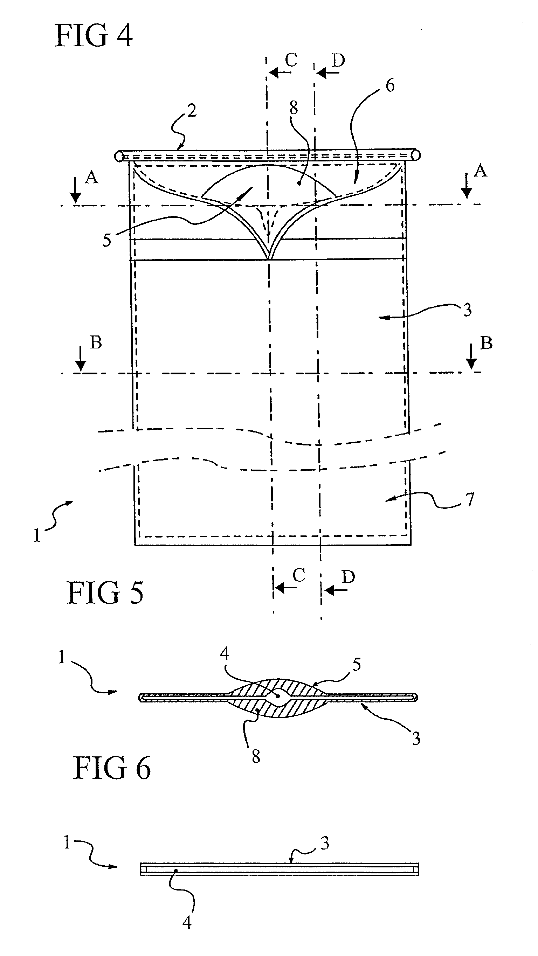

Ainsi qu'on peut le voir notamment sur les figures 3 et 4, dans cet exemple de réalisation, l'enveloppe déformable (1) est essentiellement composée d'une partie extrémale évasée (6) délimitant l'ouverture (2) et destinée à l'insertion de la poche souple (100), ainsi que d'une partie principale (7) sensiblement plus plate et destinée à contenir ladite poche souple (100) une fois introduite. Dans ce mode particulier de réalisation choisi uniquement à titre d'exemple, c'est toute la partie évasée (6) qui est renforcée puisqu'elle est conformée de manière à venir épouser les moyens de fermeture (54) lorsque ces derniers obturent notamment l'ouverture (53) de l'enceinte (52).

Selon une particularité de l'invention, chaque portion de paroi (8) qui est destinée à enserrer le conduit d'écoulement (102) lorsque la poche souple (100) est insérée à l'intérieur de l'enveloppe déformable (1), comporte une zone de renfort (5). Il est vrai que ces portions spécifiques de l'enveloppe déformable (1) sont particulièrement exposées aux risques d'usure, dans la mesure où elles subissent des frottements aussi bien extérieurement qu'intérieurement. Leurs surfaces externes et leurs surfaces internes coopèrent en effet par contact avec respectivement les moyens de fermeture (54) et avec le conduit d'écoulement (102) de la poche (100). Cette caractéristique avantageuse est particulièrement visible sur la figure 5, notamment en comparaison avec la section classique de la partie principale (7), qui est représentée à la figure 6.

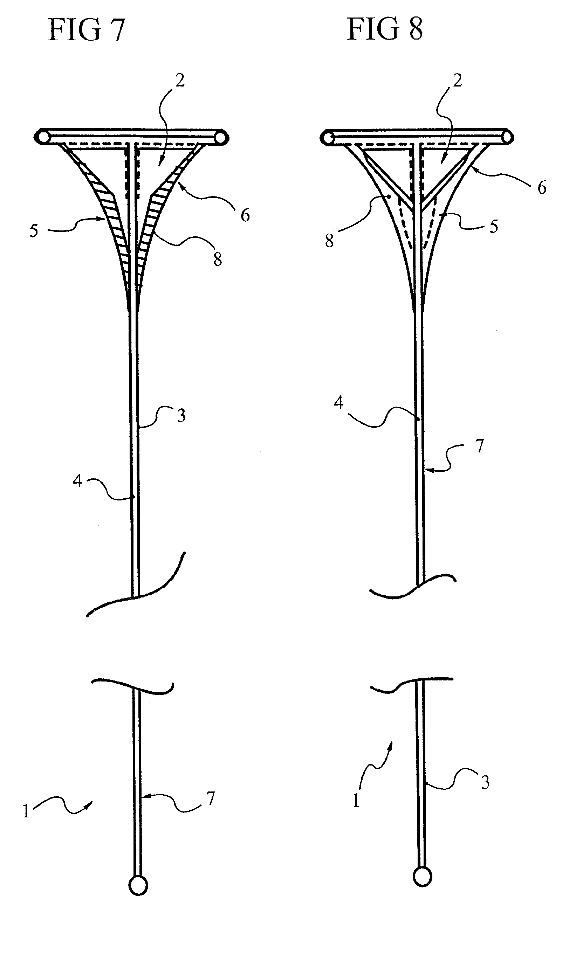

Conformément aux figures 5, 7 et 8 notamment, l'épaisseur de chaque zone de renfort (5) correspond sensiblement à deux à cinq fois l'épaisseur de la paroi du reste de l'enveloppe déformable (1).

Selon un mode de réalisation actuellement préféré de l'invention, la poche déformable (1) est réalisée en élastomère, et notamment en EPDM. Mais bien évidemment, tout autre matériau ayant des qualités de souplesse équivalentes pourrait être utilisé.

L'invention concerne également tout dispositif d'injection sous pression (50) d'un liquide (101) conditionné en poche souple (100), comportant au moins une enveloppe déformable (1) telle que précédemment décrite.

Par principe, le dispositif réclame une enveloppe déformable, mais dont la rigidité et l'épaisseur sont compatibles avec la déformation désirée. Cette rigidité est localisée aux endroits non susceptibles de se déformer, mais participe à la bonne tenue mécanique de l'ensemble, ainsi qu'à sa résistance à l'usure.

## Revendications

1. Enveloppe déformable (1) pour un dispositif d'injection sous pression (50) d'un liquide (101) conditionné en poche souple (100), destinée à s'étendre à l'intérieur d'une enceinte (52) qui, d'une part, comporte une ouverture (53) apte à être obturée par des moyens de fermeture (54) formant couvercle, et qui, d'autre part, contient un liquide inerte (51) susceptible d'être mis en pression entre la surface interne (55) de l'enceinte (52) et la surface externe (3) de l'enveloppe déformable (1), ladite enveloppe déformable (1) comportant une ouverture (2) destinée à être solidarisée hermétiquement à proximité de l'ouverture (53) de l'enceinte (52) et à être obturée par les moyens de fermeture (54), **caractérisée en ce que** chaque portion de l'enveloppe déformable (1), qui est destinée à entrer en contact avec les moyens de fermeture (54), comporte une zone de renfort dont l'épaisseur est sensiblement supérieure à l'épaisseur du reste de ladite enveloppe déformable (1).

2. Enveloppe déformable (1) selon la revendication 1, **caractérisée en ce que** chaque portion de paroi qui est destinée à enserrer un conduit d'écoulement (102) lorsqu'une poche souple (100) est insérée à l'intérieur de ladite enveloppe déformable (1), comporte une zone de renfort (5).

3. Enveloppe déformable (1) selon l'une des revendications 1 ou 2, **caractérisée en ce que** l'épaisseur de chaque zone de renfort (5) est sensiblement deux à cinq fois supérieure à l'épaisseur de la paroi du reste de ladite enveloppe déformable (1).

4. Enveloppe déformable (1) selon l'une des revendications 1 ou 3, **caractérisée en ce qu'**elle est réalisée en élastomère, notamment de type EPDM.

5. Dispositif d'injection sous pression (50) d'un liquide (101) conditionné en poche souple (100), **caractérisé en ce qu'**il comporte au moins une enveloppe déformable (1) selon l'une quelconque des revendications précédentes.
